# EUROPEAN PATENT SPECIFICATION

(11) **EP 1 099 103 B1**
(45) Date of publication and mention of the grant of the patent: **20.07.2011**
(21) Application number: 99935858.3
(22) Date of filing: 21.07.1999
(51) Int. Cl.: G01N 33/00, G01N 33/22

(54) **APPARATUS FOR DETERMINING ODER LEVELS IN GAS STREAMS**
GERÄT ZUR BESTIMMUNG DER GERUCHSNIVEAUS IN GASSTRÖMEN
APPAREIL PERMETTANT DE DETERMINER DES NIVEAUX D'ODEUR DANS DES FLUX GAZEUX

(30) Priority: 23.07.1998 US 121512; 03.02.1999 US 244560
(43) Date of publication of application: 16.05.2001
(73) Proprietor: MILTON ROY COMPANY, Ivyland, PA 18974-0577 (US)
(72) Inventor: MARSHALL, Stephen, Edward, Conroe, TX 77302 (US); SELMAN, Glenn, Scott, Spring, TX 77388 (US); SKUBIS, Christopher, Lee, The Woodlands, TX 77380 (US)
(74) Representative: Leckey, David Herbert
(86) International application number: PCT/US1999/016634
(87) International publication number: WO 2000/005563

(56) References cited:
- FR-A- 2 210 297
- US-A- 3 882 713
- US-A- 5 675 070
- US-A- 5 767 385
- U. LACHENMAYER ET AL: "Untersuchungen zur Neuentwicklung eines Olfaktometer" STAUB-REINHALTUNG DER LUFT, vol. 44, 1984, pages 359-362, XP002289210 DE

## Description

### Technical Field

The present invention relates generally to apparatus for determining odor levels in odorized gas streams.

### Description of the Related Art

Federal and state regulations require that combustible gases (e.g., natural gas) in transmission lines be odorized to provide a quick and easy means for detecting the presence of gases. Odorization involves adding small amounts of a chemical with a distinctive odor to the gas stream. This odor facilitates leak detection and provides an early warning of potentially unsafe conditions.

The odor must be readily detectable by a person with a normal sense of smell when gas is present at a given concentration in air. The concentration is typically specified as a certain percentage of the lower explosive limit (LEL) of the gas.

Federal and state odorizing regulations require gas operators to conduct periodic sampling at distribution sites to assure proper odor levels. Portable instruments, sometimes referred to as odorometers, have been devised to determine gas odor levels using olfactory testing. These instruments are available from a number of manufacturers including Bacharach, Inc. and Heath Consultants, Inc.

In one known device, gas to be analyzed enters the instrument through a gas intake port. The gas passes through a low pressure regulator and then a flow adjustment valve, which is operated by a user. The gas then enters a sensor chamber, which measures gas flow. A signal from the sensor chamber is processed, converted to a digital signal and fed to a liquid crystal display (LCD). The gas from the sensor chamber is then mixed with air drawn in by an air blower. The air-gas mixture then exits the device through an exhaust port, where it can be sniffed by a user.

One test that is commonly performed with such a device is known as a threshold odorant test (TOT). This test is used to determine a threshold gas-air concentration at which odor is barely detectable. In this use, the operator slowly opens the flow adjustment valve, allowing the gas sample to enter the instrument, while at the same time breathing normally with his or her nose placed close to the exhaust port. When odor in the sample is detected, the operator depresses a read button to observe a reading on the LCD corresponding to gas flow. A look-up chart is then used to convert the reading into a percent gas concentration, which is then hand-written somewhere for recordation.

Another use of the device is to determine odor characteristics at given gasair concentrations. In this use, a gas/air mixture at a preset concentration is sniffed by various persons who then categorize the odor level as "absent," "barely detectable," "readily detectable," "strong" or "obnoxious."

A significant problem with these known devices it that they fail to provide accurate and repeatable gas concentration readings. Except for the LCD output, the instruments are substantially analog devices and lack adequate control means to ensure steady operation. For instance, the fans or blowers used to draw air through the devices cannot be sufficiently controlled to ensure constant speed, which is critical in determining gas concentration.

Furthermore, the known devices fail to provide adequate mixing of the sample gas and air. Consequently, the gas-air mixtures are not uniformly mixed, resulting in testing inaccuracies.

In addition, known devices are not equipped with recording means for storing and/or analyzing test data. Consequently, users must hand-write the data somewhere and use a look-up table to cross-reference data with gas concentration values. This is a potential source for further errors.

Apparatus having the features of the preamble of claim 1 is disclosed in FR-A-2210297. An automated defactometer is disclosed in US-A- 5767385.

### BRIEF SUMMARY OF THE INVENTION

The device disclosed herein includes a mixing chamber for mixing gas from a gas supply to be tested with air to form a gas-air mixture. The mixing chamber includes a gas inlet port through which the gas is received, an air intake port through which the air is received, and an outlet port through which the gas-air mixture exits the mixing chamber. A static mixing element is located at the air intake port to cause turbulence in incoming air flow to promote thorough mixing of the air and gas. The device includes a flow control valve that is connected to the gas supply to enable a user to selectively adjust the flow rate of the gas entering the device. A mass flow sensor is positioned between the flow control valve and the gas inlet port of the mixing chamber for accurately measuring the mass flow rate of the gas flowing into the mixing chamber. A motorized fan is positioned in a hose connected to outlet port of the mixing chamber for drawing the gas-air mixture out of the mixing chamber and moving it to a sniff chamber, where it can be sniffed by the user to detect odor.

The device is equipped with a microprocessor controller including a tachometer for determining the rotational speed of the motorized fan. The microprocessor controller is responsive to signals from a fan speed sensor for controlling the rotational speed of the motorized fan such that it is maintained at a substantially constant speed. Consequently, there is substantially constant air flow into the device.

A rechargeable lead-acid battery is provided for powering the instrument.

The device includes memory for storing test data. It is equipped with a keypad and an LCD display for user interface.

The device displays test results in actual air/gas percentages, eliminating the need for look-up charts.

The device records complete test results electronically. It can also record any comments by technicians relating to a given test such as, e.g., information on weather conditions at a test site. In addition, test records are stamped with date/time information as well as information on device calibration.

The apparatus can be linked to a PC to enable test and device data to be exchanged between the PC and the apparatus. A software package is provided for the PC to generate reports, graphs, and data archives from data read from the device. The device thus eliminates the need to manually write test reports. In addition, critical test data recorded by the device cannot be altered using the device. This feature substantially reduces the risk of test data being falsified by technicians.

Test data downloaded from the device to a PC can also be exported to third-party developed applications for further processing.

In addition, the software package allows user, test location and calibration information to be easily uploaded from the PC to the device. This feature eliminates the need for technicians to prepare forms and perform repetitive data entry.

The device also includes a user authentication feature. Each user is required to enter an identification and corresponding password before the device can be used.

### BRIEF DESCRIPTION OF THE DRAWINGS

For a more complete understanding of the present invention and the advantages thereof, reference should be made to the following Detailed Description taken in connection with the accompanying drawings, in which:
**Figure 1** is an exploded perspective view of a device for determining odor levels in accordance with the invention;
**Figure 2** is an exploded perspective view of the main unit assembly shown in the **Figure 1**;
**Figure 3** is an exploded perspective view of the mounting panel assembly of the main unit assembly;
**Figure 4** is an exploded perspective view of the fan sub-assembly of the main unit assembly;
**Figure 5** is an exploded perspective view of the fan printed circuit board assembly of the fan sub-assembly;
**Figure 6** is a schematic diagram illustrating gas and air flow through the device;
**Figure 7** is a schematic block diagram illustrating microprocessor control of the device;
**Figure 8** is a high-level state diagram illustrating operation of the device;
**Figure 9** is a high-level reporter state diagram illustrating operation of PC software for analyzing test data from and for programming the device;
**Figure 10** is an exemplary screen shot generated by the PC software of a test log;
**Figure 11** is an exemplary screen shot generated by the PC software illustrating exporting of data to external applications;
**Figure 12** is an exemplary screen shot generated by the PC software illustrating data filtering;
**Figure 13** is an exemplary screen shot generated by the PC software of a graph of test readings at a given test location;
**Figure 14** is an exemplary screen shot generated by the PC software showing details of a given test conducted using the device;
**Figure 15** is an exemplary screen shot generated by the PC software illustrating pre-loading of user and test location data on the device;
**Figure 16** is an exemplary screen shot generated by the PC software showing details of a qualification test taken by a given user; and
**Figure 17** is an exemplary screen shot generated by the PC software of a graph of the results of multiple qualification tests taken by a given user.

### DETAILED DESCRIPTION OF THE PREFERRED EMBODIMENT

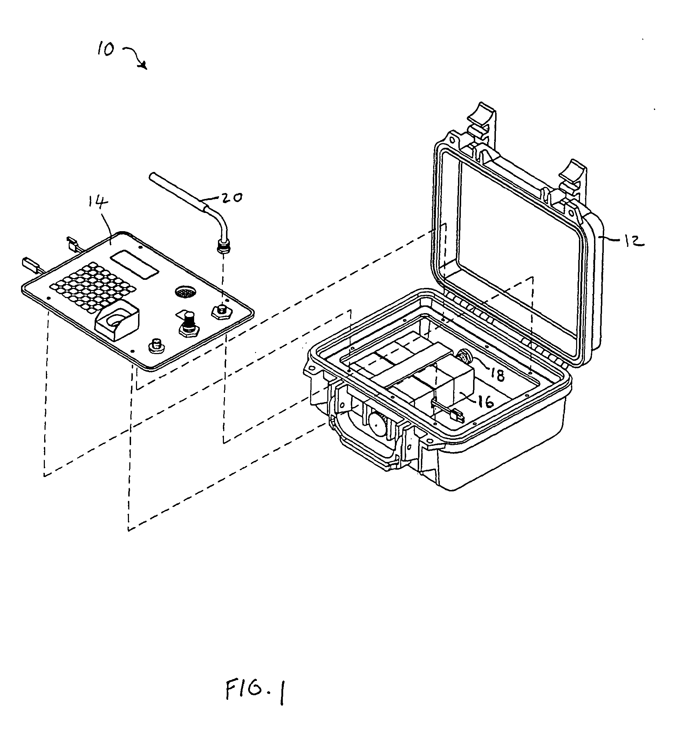

**Figure 1** is an exploded view of an odorometer device or instrument **10** for correlating odor levels in gas streams with gas in air concentrations in accordance with the invention. The device **10** includes a portable outer housing or case **12.** The case **12** is equipped with a main unit **14** and a battery **16** for powering the unit. The battery **16** is preferably a rechargeable lead-acid battery. A charger port **18** is provided in the case **12** so that the battery **16** can be charged in situ. Use of a battery facilitates portability of the device, though it should be noted that the device can be configured to be AC powered if desired.

An inlet hose **20** is optionally included to facilitate coupling of the instrument with a gas supply or source (not shown in **Figure 1**) to be tested. A variety of odorized gases can be tested including, e.g., natural gas and liquefied petroleum gas such as propane and butane.

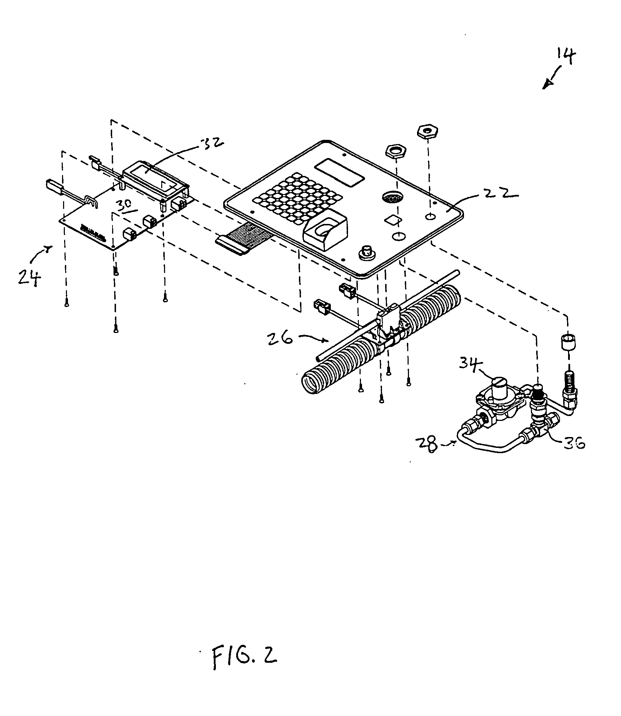

**Figure 2** is an exploded view showing the main unit assembly **14** in greater detail. The main unit assembly **14** includes a mounting panel assembly **22,** a main printed circuit board (PCB) assembly **24,** a fan sub-assembly **26,** and a regulator assembly **28.**

The main PCB assembly **24** includes a processor board **30** containing the microprocessor circuitry for controlling and operating the instrument as will be described further below. The main PCB assembly **24** also includes a 4 x 20 character LCD display **32.**

The regulator assembly **28** comprises a low flow regulator **34** for regulating the pressure of gas from the gas supply. It also includes a flow control valve **36,** preferably a precision needle valve, that can be adjusted by a user to control the flow rate of gas from the gas supply.

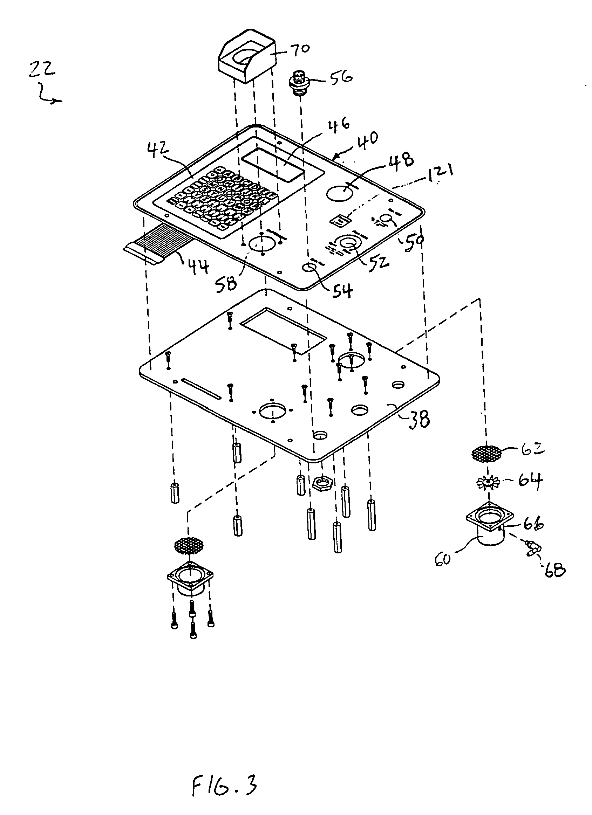

**Figure 3** illustrates the mounting panel assembly **22** in greater detail. The mounting panel assembly **22** comprises a base panel **38** and an overlay panel 40. The overlay panel 40 includes a membrane keyboard or keypad **42** having a 7x7 matrix. A connector cable **44** from the keypad **42** connects to the main PCB assembly **24.** The base and overlay panels **38, 40** include a window **46** through which the LCD display **32** can be viewed. It also includes an air intake port **48,** a gas inlet port **50,** an opening **52** for receiving an adjustment knob of the flow control valve **36,** an opening **54** for receiving a data port connector **56,** and a gas outlet port **58.**

The air intake port **48** leads to a mixing chamber **60** directly therebeneath. A screen **62** and a mixing element **64,** preferably comprising a static (i.e., nonrotating) fan, are positioned at the air intake port **48.** It should be noted that other types of mixing elements can alternatively be used, e.g., a series of baffles. The mixing chamber **60** includes a gas entry port **66** through which gas enters the mixing chamber at a location slightly below the fan element **64.** A polyethylene elbow tube **68** supplies gas to the gas entry port **66.**

A nose piece **70,** which forms a "sniffing chamber" at which gas is sniffed by a user, is connected to the outlet port **58.**

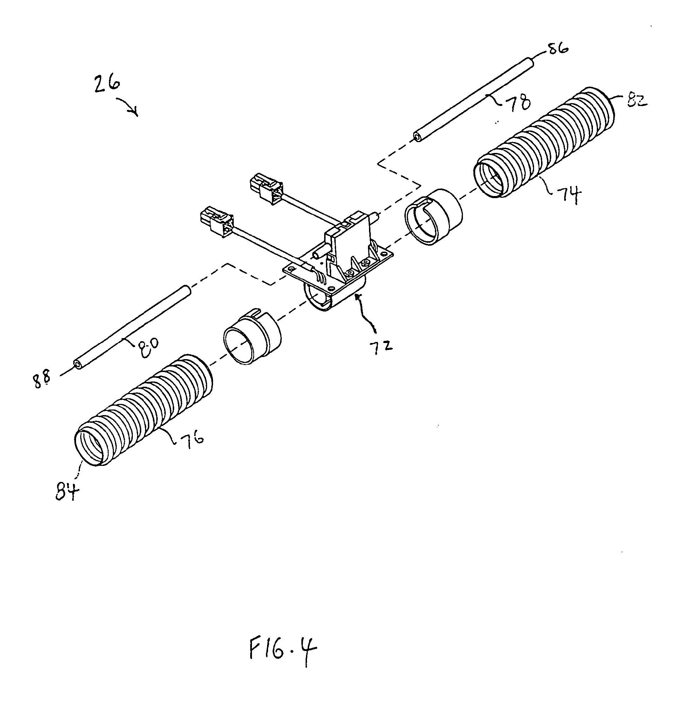

**Figure 4** is a detailed exploded view of the fan sub-assembly **26** (of **Figure** 2). The fan sub-assembly **26** comprises a fan PCB assembly **72,** which is connected on opposite sides by first and second conduits or hoses **74, 76** and first and second tubes **78, 80.**

The hoses **74, 76** are preferably flexible polypropylene hoses, each having a length of four inches and a one-inch inner diameter. The inlet end **82** of the first hose **74** connects directly with the mixing chamber **60** to receive flow therefrom. The outlet end **84** of the second hose **76** connects directly with the gas outlet port 58 for exhausting gas from the device **10.**

The tubes **78, 80** are preferably of quarter-inch outer diameter, each having an eight-inch length. The inlet end **86** of the first tube **78** is connected to the outlet of the flow control valve **36.** The outlet end **88** of the second tube **80** is connected to the elbow tube 68 leading to the mixing chamber **60.**

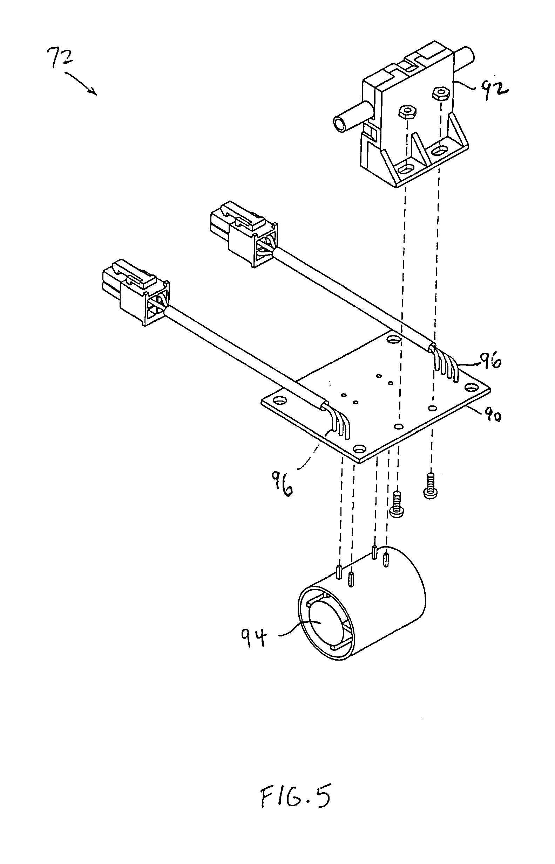

The fan PCB assembly **72** (of **Figure 4**) is shown in greater detail in **Figure 5****.** The fan PCB assembly **72** includes a printed circuit board **90,** on which a mass flow sensor **92** and a motorized fan **94** are connected. The mass flow sensor **92** is connected on opposite sides to the tubes **78, 80** to measure flow of gas therethrough. The mass flow sensor **92** transmits signals indicative of the gas flow rate through circuitry on the PCB **90** to the microprocessor controller on the main PCB assembly **24.**

Also mounted on the PCB **90** is a fan speed sensor **96,** preferably an infrared device. The sensor **96** includes an infrared emitter/receiver pair located on opposite sides of the fan **94.** The emitter sends an infrared beam to the receiver. The beam is interrupted each time a fan blade crosses it as the fan **94** rotates. A pulsed signal indicative of the fan rotation speed is thereby transmitted by the sensor **96** to a tachometer **97** in the microprocessor controller. A closed loop control circuit comprising the infrared sensor/tachometer and a digitally controlled drive circuit are used to keep the fan at a constant speed. The main controller can adjust the fan speed as necessary by outputting a signal to a digital potentiometer in the main PCB **24** to adjust the drive voltage to the fan **94.**

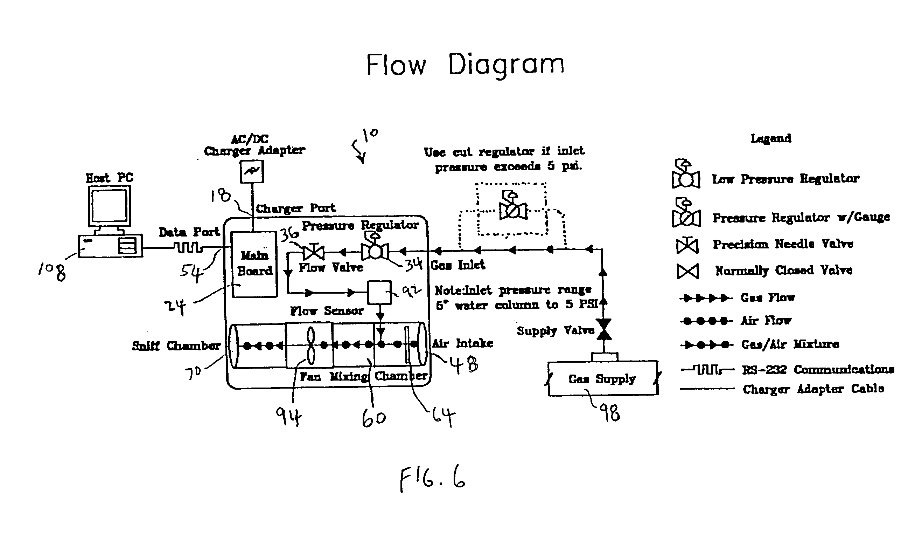

Referring now to **Figure 6****,** the path of air and gas flow through the device **10** will now be described. Gas from a gas supply **98** flows through tubing leading to the regulator **34.** The gas flows through the regulator **34** and thereafter through the flow control valve **36** (when it is open). Thereafter, gas flows through the inlet end **86** of first tube **78** and then through the mass flow sensor **92,** which determines its flow rate. Thereafter the gas flows through the second tube **80** which is connected to the elbow tube **68** leading to the mixing chamber **60,** where the gas is mixed with air.

Air enters the instrument **10** through the air inlet port **48** shown in **Figure 3****.** It flows through the screen **12** and past the stationary fan element **64** into the mixing chamber **60.** The fan element **64** is designed to swirl incoming air thereby creating turbulence to promote mixing with incoming gas. The air and gas are mixed in the mixing chamber **60** to form a mixed gas/air mixture, which flows into the inlet end **82** of the first hose **74.** The air/gas mixture is drawn through the hose **74** by the motorized fan **94** and forced through the second hose **76,** which is connected to the outlet port **58.** The gas/air mixture flows through the outlet port and out of the nose piece **70** forming the sniff chamber.

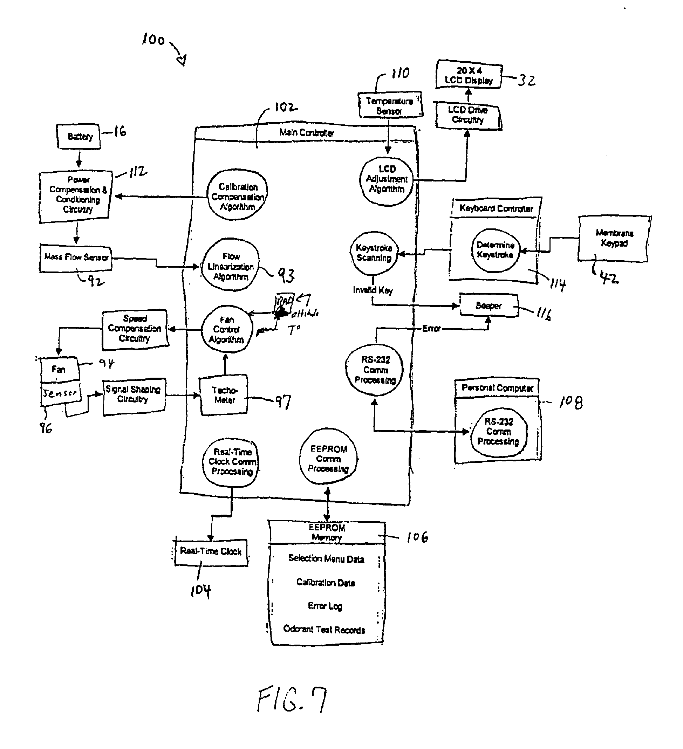

**Figure 7** is a block diagram illustrating the microprocessor circuitry **100** for controlling various components of the device **10.** The circuitry **100** includes a main controller **102,** which along with associated firmware, monitors all the system data inputs and provides control and data outputs for various system devices. An on-chip analog to digital (A/D) converter is used to convert critical analog inputs to digital signals for processing. A timer input captures the tachometer pulses from the motorized fan assembly. Digital potentiometers are used to provide voltage controlled outputs for contrast adjust (for the LCD), fan speed and flow sensor offset and span.

The device **10** also includes a real time clock chip **104** to keep current date and time information. The real time clock **104** enables test results and errors to be date and time stamped for record keeping purposes. A battery backup keeps the date and time on the clock chip **104** current when power is off.

The device **10** further includes a non-volatile memory, preferably at least one electrically erasable, programmable read-only memory chip (EEPROM) **106** to store user information, test location, test records, error logs, and calibration/parameter data. The EEPROM **106** can be uploaded to or downloaded from by a PC **108** using a software package, which will be further described below. This allows for a paperless audit trail. For convenience, user and test location information can be written to the EEPROM **106** by the PC **108** to reduce user data entry in the field.

Ambient temperature readings from an onboard temperature sensor **110** are monitored by the microprocessor controller **102** and used to automatically adjust the LCD display contrast with temperature variations. Adjustments are made using a digital potentiometer.

The device **10** also includes battery compensation and conditioning circuitry **112,** which automatically compensates for battery voltage level changes. The circuitry **112** can include, e.g., a voltage regulator. Battery compensation allows a wider range of battery voltage to be used while maintaining the calibration accuracy of the flow sensor **92** and increasing the charge life of the battery **16.**

The device **10** uses a flow linearization algorithm **93** using a multi-point calibration process of the mass flow sensor readings to provide a linear flow response. The LCD **32** can thus display gas-in-air concentrations, obviating the need for a user to cross-reference readings with a look-up table.

A keyboard controller **114** is used as a keyboard decoder. The keyboard controller **114** scans the keyboard **42** for a key press and then communicates the key press to the main controller **102.** If an invalid key is pressed, a beeper **116** is activated to notify the user of the error.

The device **10** can be linked to the PC **108** through the data port **54.** The PC 108 includes a software package used as an interface tool to allow uploading and downloading of data to/from the device **10.** Stored test records, user information, test location information, and error logs can be read from the device and used to generate reports, graphs and data archives. Information on users, test locations, and device calibration can be uploaded from the PC **108** to the device **10.**

The device **10** can also be configured to be linked through the data port **54** to a printer for printing data stored in the device.

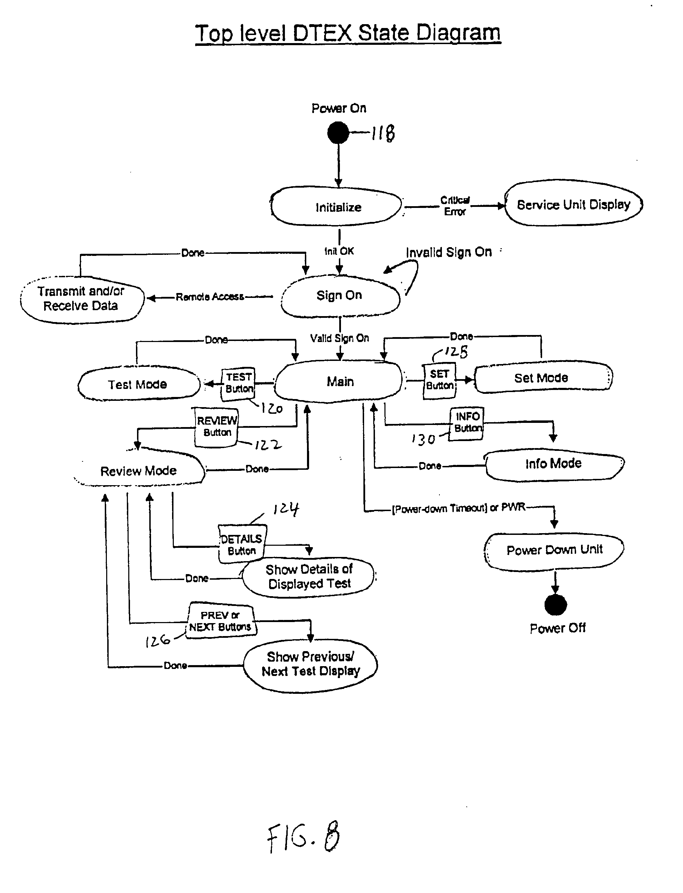

**Figure 8** is a state diagram illustrating operation of the device **10** through keypad input. (The term "DTEX" used in the drawings is a trademark for the device **10.)** Initially the user presses the "Power On" button **118** on the keypad **42** causing the system to initialize. If an error occurs during the initialization process, a "Service Unit" message is shown on the LCD **32.** After the device **10** has been initialized, the user can (if desired) connect the device through the data port **54** to the PC **108** in order to transmit and/or receive data.

Thereafter, the user is prompted to "sign on," and after a valid sign on, the main mode is entered. The device includes a user authentication feature for security. For a valid sign on, each user is required to input identification information and a corresponding password.

If the user presses the "Test" button **120,** the device **10** switches to the test mode and can be used to conduct testing. For example, threshold odor testing can now be conducted as follows. First, the user enters any needed information on the keypad, including, e.g., information identifying the gas supply location. Next, the inlet hose **20** is connected to the gas supply to be tested. Then, with the fan **94** having been turned on, the user gradually opens the gas flow valve **36,** allowing the gas sample to enter the instrument **10.** At the same time, the user breathes normally with his or her nose placed at the nose piece **70.** When odor in the sample is detected, the user depresses a "Record Test Level" button **121** (shown in **Figure 3****).** The gas concentration reading is displayed on the display **32** and stored in device memory **106.**

The user can enter any comments he or she may have relating to the test such as, e.g., wind conditions at the test site.

The user can press a "Review" button **122** to place the device **10** in a review mode, in which test results can be reviewed. If the "Details" button **124** is pressed, specific details of a particular test are displayed. The user can also press "Previous" or "Next" buttons **126** to scroll through other test information. While the user can review test data, the device **10** is programmed to prevent the user from altering critical test data in order to prevent any attempt to falsify data.

The "Set" button **128** can be pressed to set up new users and to set the system clock.

The "Info" button **130** can be pressed to switch to an information mode to, for example, look at the serial number of the unit, calibration data of the unit or the last reported error of the unit.

The device **10** can be powered down by the user or will automatically power down if no request has been made within a given period of time, for example, thirty minutes.

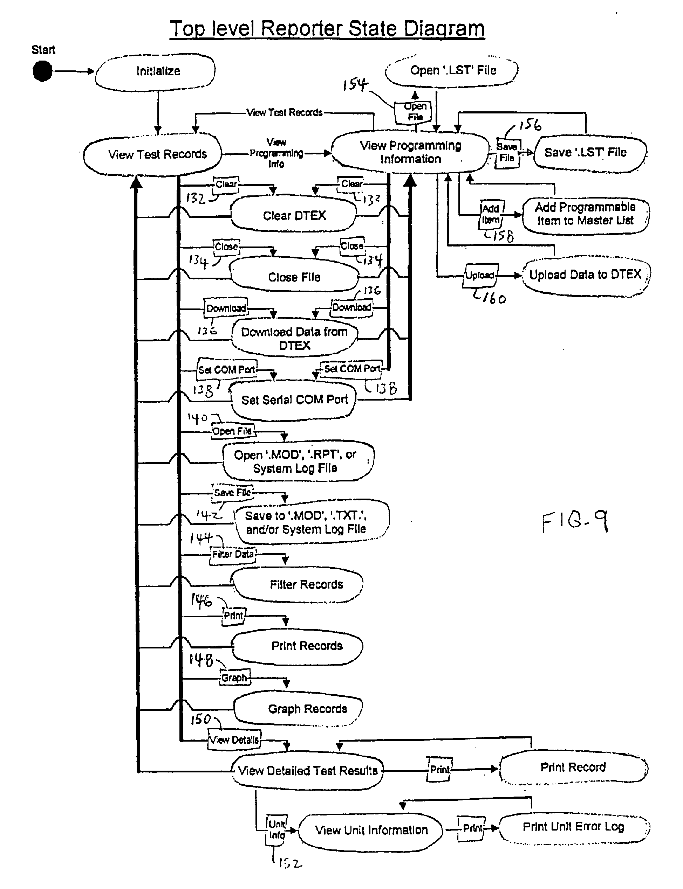

After all the desired readings have been taken of gas samples, the instrument can be taken to a central location site and data stored therein can be downloaded to a PC **108.** In accordance with the invention, Windows-based software is provided for the PC to prepare test data into formal reports needed to satisfy the formal reporting requirements. The data from the device can also be logged onto a main data base and retrieved as needed. The software also enables devices to be programmed. **Figure 9** is a high level state diagram illustrating use of the PC **108** for uploading and downloading data to/from a selected device once it has been linked to the PC **108** through the data port **54.** After initialization, a user can view test records and programming information on the PC display. The software provides the user with several options for managing test records and device programming information. The user can use a pointing device such as a mouse to move a cursor on the PC display to select and click on various items. A "Clear" button **132** can be pressed to clear the memory (EEPROM) on the device 10. A "Close" button **134** can be pressed to close any opened file. A "Download" button **136** can be pressed to download data from the device memory. A "Set COM Port" button **138** can be pressed to change the serial COM port as desired.

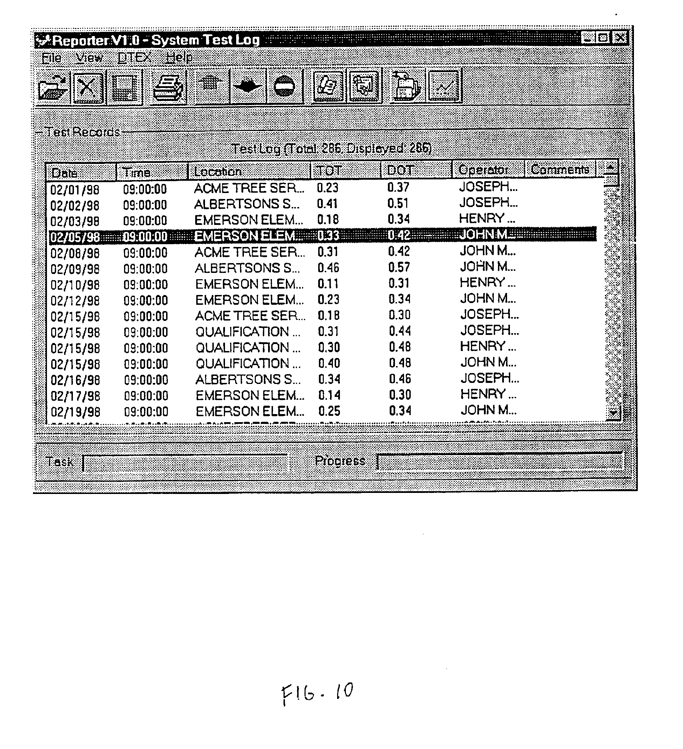

An "Open File" **140** button can be pressed to open one of three different types of files. The ".MOD" files mirror the files stored in the device. The ".RPT" file contains an archive of test results. The "System Log" file is a master database of all tests run at all sites. **Figure 10** is an exemplary screen shot generated by the PC software of a test log.

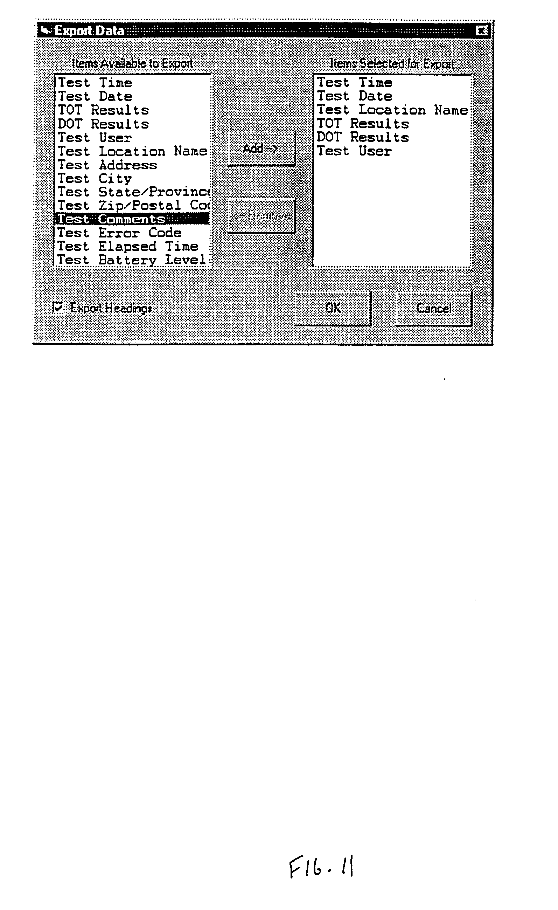

The "Save File" button **142** can be pressed to save test records in different file types, including ".TXT" files, which can be e-mailed if desired. The files can also be imported into third party developed software. **Figure 11** is an exemplary screen shot illustrating exporting of data to such third party applications.

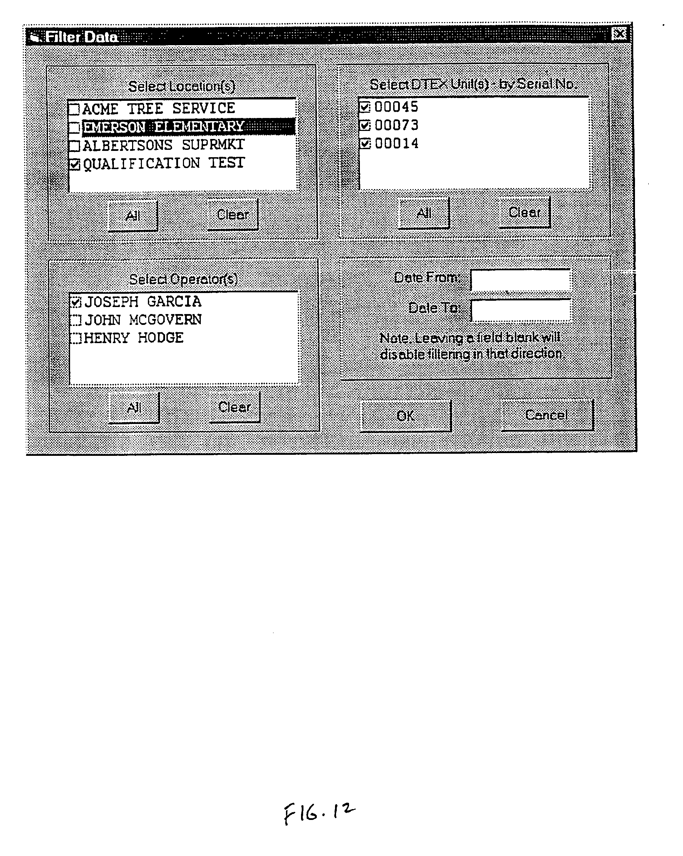

A "Filter Data" button **144** can be pressed to allow stored records to be filtered (i.e., queried) by, e.g., test date, user name, test location and particular unit used. **Figure 12** is an exemplary screen shot illustrating data filtering.

A "Print" button **146** can be pressed to print a file.

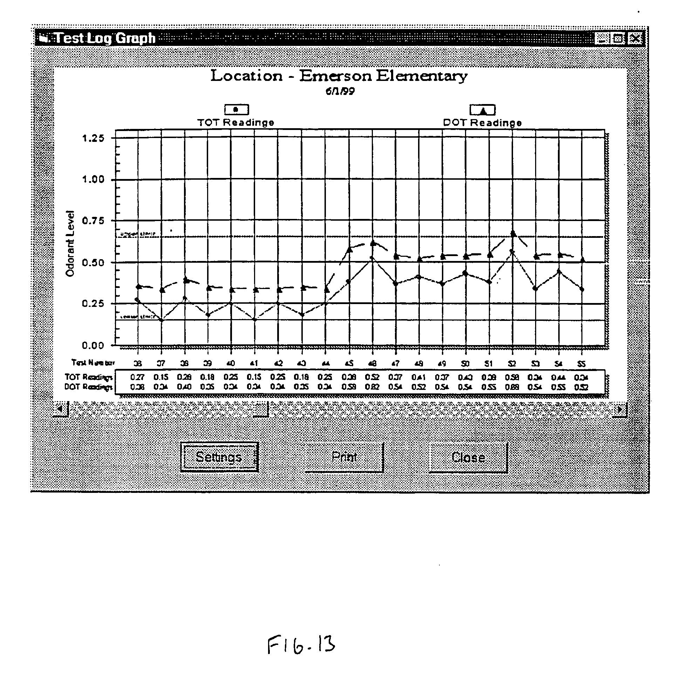

A "Graph" button **148** can be pressed to graph test results. **Figure 13** is an exemplary screen shot of a graph generated from test readings at a given test location.

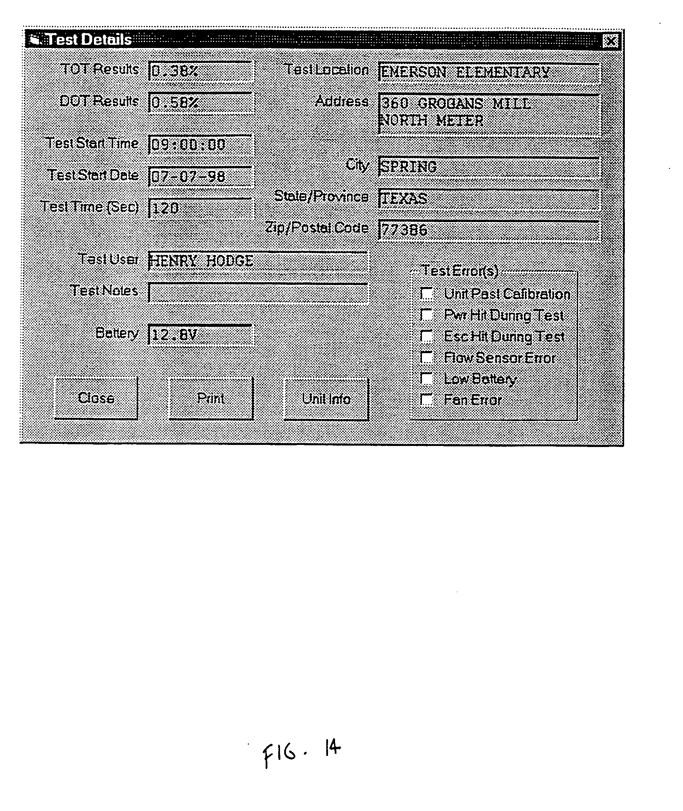

A "View Details" button **150** can be pressed if the user wishes to see complete data of a given test. **Figure 14** is an exemplary screen shot showing details of a given test conducted using the device.

A "Unit Information" button **152** can be pressed to view critical parameters of the device, e.g., calibration and error log data.

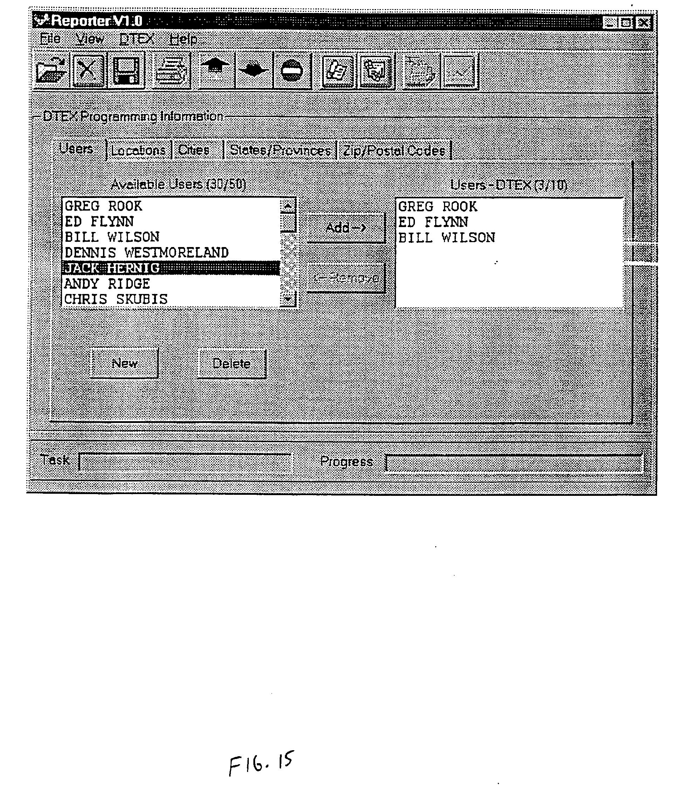

In the "View Programming Information" mode, the user can press an "Open File" button **154** to view a list of all files that have been uploaded to or downloaded from the device. **Figure 15** is an exemplary screen shot illustrating pre-loading of user and test location data on the device.

By pressing a "Save File" button **156,** a user can save programming information including, e.g., configuration information. This information can be used to program another device in the same way as the subject device, which may, e.g., be in need of service.

The user can press an "Add Item" button **158** to add a programmable item to the master list such as, e.g., a new user and/or location. The user can also press an "Upload" button **160,** which allows data to be uploaded to the device.

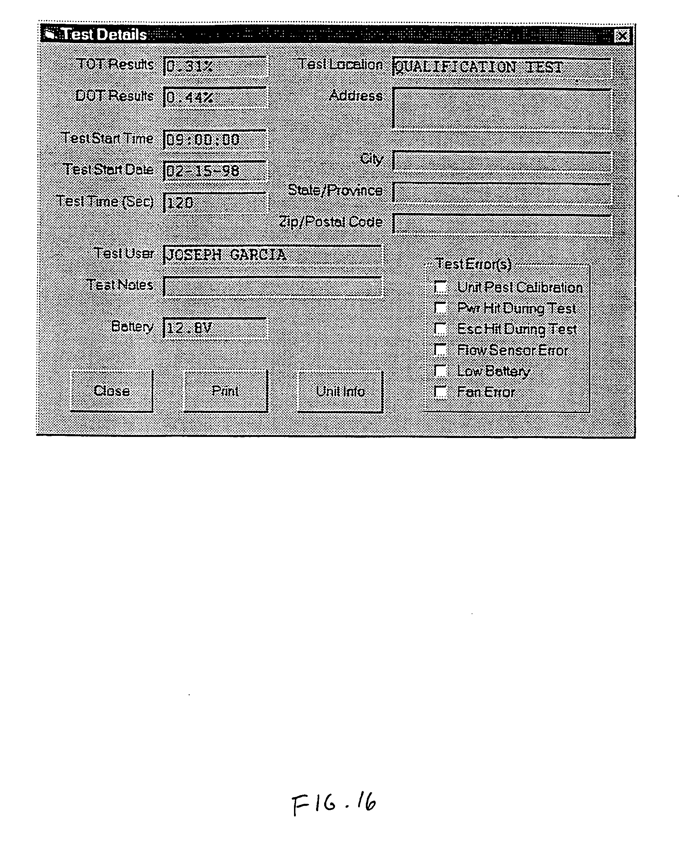

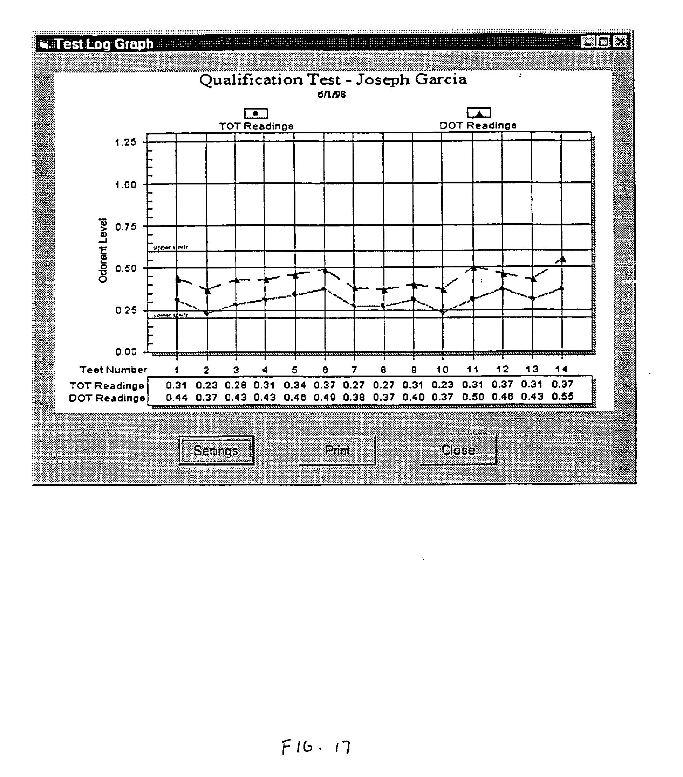

The device **10** can be used to test the smell sensitivity of technicians working in the industry since individuals will have varying sensitivity to odor. The automatic data recording feature helps prevent any test cheating by users. Figure **16** is an exemplary screen shot showing details of a qualification test taken by a given user. **Figure 17** is an exemplary screen shot of a graph of the results of multiple qualification tests taken by a given user.

The software preferably comprises a set of instructions in a code module resident in a random access memory of the PC **108.** Until required by the computer, the set of instructions may be stored in another computer memory such as a hard disk drive or in a removable memory such as an optical disk for eventual use in a CD ROM or a floppy disk for eventual use in a floppy disk drive, or even downloaded via the Internet.

In addition, although the various methods described are conveniently implemented in a general purpose computer selectively activated or reconfigured by software, one of ordinary skill in the art would also recognize that such methods may be carried out in hardware, in firmware, or in more specialized apparatus constructed to perform the method steps.

As has been described, the present invention provides a microprocessor-based odorometer wherein a motorized fan is maintained at a substantially constant speed to facilitate a substantially constant air flow into the sniff chamber. A further enhancement to the inventive approach is now described. In particular, when the apparatus is used at high altitudes, the fact that there is lower air density means that less air is provided to the blower. In addition, variations in ambient temperature also impact air density. Thus, according to the present invention, in conjunction with the fan motor speed correction, the microprocessor-based control system also accounts for air density variations (whether caused by altitude or temperature variations, or both). In general, when and where lower air density is encountered, the fan speed is adjusted upwards. On the contrary, when and where higher air density is encountered, the fan speed is adjusted downwards. In either case, the goal of maintaining a constant air flow is maintained.

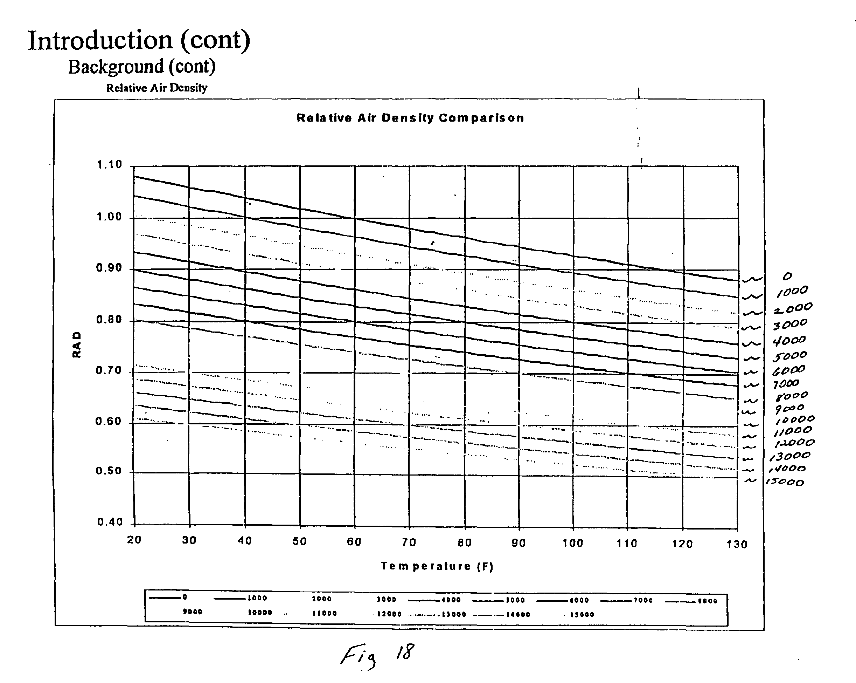

Thus, according to an alternate embodiment of the present invention, a target fan speed is established upon initiation of the test as a function of relative air density at the given location where the test is being undertaken. In particular, during initialization, the user may enter a new location **9** or recall a stored location) at which the test is being performed. One of the given fields of information that may be entered about the location is the altitude of the location. Another field of information is the ambient temperature. The keypad is used to enter such information. With the altitude and temperature information, the microprocessor (during setup) calculates a target fan speed by multiplying a given fan speed by an adjustment factor. Preferably, the adjustment factor is a Relative Air Density (RAD) that is obtained from a look-up table stored in the device. (Alternatively, the look-up table may be evaluated manually by the user, with the RAD factor then input through the keypad). **Figure 18** is a table illustrating a Relative Air Density Comparison for given elevations at given temperatures. Using the elevation and temperature inputs, an RAD value is computed (or manually entered). That value is then multiplied by the given fan speed to derive the target fan speed. The controller then drives the fan motor either at a higher speed or lower speed as compared to the given fan speed, but still maintains the constant air flow that is desired.

Accounting for changes in air density provides significantly-enhanced operation of the device. The apparatus may be taken to various locations at various elevations yet still provide consistent and accurate readings across all tests. By applying a relative air density compensation to the fan speed motor, the test results fully account for variations in altitude and temperature. The device thus enables the operator to accurately quantify the gas concentration in air irrespective of where the test is being performed, all with a single integrated device. Operators of such devices are assured of consistent, useful results, despite variations in testing conditions.

As noted above, the RAD compensation is typically effected only once, namely, before initiation of the actual sniff test. One of ordinary skill in the art will appreciate, however, that dynamic adjustments to the fan motor target speed may be provided as a function of ambient conditions that might change during the test. Further, it may be desirable to further compensate fan motor speed to take into consideration changes in barometric pressure (e.g., due to changing weather conditions across a set of tests). With a microprocessor-based controller, such modifications and variations may be readily implemented.

Thus, according to the present invention, the fan delivers an appropriate amount of air (for the sniff test) and the controller holds the fan speed constant, irrespective of the altitude and/or temperature conditions at the location where the device is being used. This is accomplished by normalizing the air flow control algorithm (i.e., the algorithm that controls the fan motor speed) to temperature and altitude variations through the RAD factor. If desired, the fan motor speed may only be normalized as a function of temperature or as a function of altitude, but improved results are provided when both factors are considered.

As noted, the particular method by which the temperature or altitude inputs are provided is not restricted to any give approach. In the preferred embodiment as discussed above, the calibration is performed in response to input of both values, but this is not a limitation. The system may store a set of elevations cross-referenced to given locations, or the user may enter the RAD manually after calculating the necessary value from the table in **Figure 18****.**

As a corollary, the various interface screens and output reports previously described preferably also include appropriate fields identifying the altitude and temperature data.

Having thus described my invention, what I claim as new and desire to secure by Letters Patent is set forth in the following claims.

## Claims

1. A portable apparatus (10) for determining odor levels in gas streams comprising:
a mixing chamber (60) for mixing gas from a gas supply with ambient air to form a gas-air mixture said mixing chamber (60) including a gas inlet port (66) through which the gas is received an air intake port (48) through which the air is received, and an outler port through which the gas-air mixture exits said mixing chamber;
a valve (36) connectable to the gas supply enabling a user to selectively adjust flow of the gas from the gas Supply to the apparatus (10): and
a mass flow sensor (92) positioned between the flow control valve (36) and the gas inlet port (66) of said mixing chamber (60) for measuring the mass flow rate of the gas flowing into the mixing chamber (60). **characterised in that** the apparatus further comprises:
a motorized fan (94) for moving the gas-air mixture out of the mixing chamber (60) to a location where said gas-air mixture can be sniffed by the user to detect odor;
a controller (102) for maintaining said motorized fan (94) at a substantially constant given speed;
a processor:
a keypad (42) for entry of data related to a given test;
a display (32) displaying data related to a given test:
a non-volatile memory (106) operably coupled to the processor for storing the data related to the given test, the data including one or more of the following: an odor detection level, an operator identifier, a dare and a timestamp of the test, a test location, a test time, operator-entered notes, and an indication of any test errors; and
program means executable by the processor for restricting access by a user to the data stored in the non-volatile memory.

2. The portable apparatus of claim 1 wherein said controller (102) includes a tachometer (97) for determining rotational speed of me motorized fan (94).

3. The portable apparatus of claim 1 or 2 further comprising an infrared emitter and receiver pair (96) positioned on opposite sides of said motorized fan (94) for generating an infrared beam that is interruptable by movement of blades of said motorized fan (94).

4. The portable apparatus of claim 1, 2 or 3 further comprising a mixing element (64) at said air intake port (48) of said mixing chamber (60) for causing air flow turbulence to promote mixing of said air and gas.

5. The portable apparatus of claim 4 wherein said mixing element (64) comprises a stationary fan.

6. The portable apparatus of any preceding claim wherein said motorized fan (94) is located in a conduit connected to the outlet port of the mixing chamber (60) for drawing the gasaiF mixture out of said mixing chamber.

7. The portable apparatus of any preceding claim further comprising means for linearizing mass flow rate data generated by the mass flow sensor (92).

8. The portable apparatus of any preceding claim further comprising a battery (16) for powering the apparatus.

9. The portable apparatus of claim 8 wherein said battery (16) is rechargeable.

10. The portable apparatus of claim 9 further comprising a charger port connected to said battery (16) for enabling said bakery to be recharged in situ.

11. The portable apparatus of claim 10 further comprising barrery compensation circuitry (112) for enabling said mass flow sensor (92) to operate accurately within a given voltage range of said battery (16).

12. The portable apparatus of any preceding claim further comprising means for linking said apparatus (10) to a computer (108) for transmining data to and receiving data from said computer.

13. The portable apparatus of any preceding claim further comprising means for linking said apparatus (10) to a printer for printing data from said apparatus.

14. The portable apparatus of claim, wherein the computer (108) includes means for processing and displaying the data related to the given test.

15. The portable apparatus of claim 14, wherein said means for processing data include means for generating graphs from said data.

16. The portable apparatus of claim 14, wherein said means for processing data include means for filtering said data.

17. The portable apparatus of claim 12 wherein said computer includes means for archiving the data related to the given test.

## Patentansprüche

1. Tragbare Vorrichtung (10) für die Bestimmung von Geruchs-Pegeln in Gasströmen, aufweisend:
eine Mischkammer (60) zum Mischen von Gas von einer Gaszufuhr mit Umgebungsluft, um ein Gas-/Luft-Gemisch zu bilden, wobei die Mischkammer (60) eine Gaseinlassöffnung (66), durch die das Gas eingeleitet wird, eine Lufteinlassöffnung (48), durch die die Luft eingelassen wird, und eine Auslassöffnung aufweist, durch die das Gas-/Luft-Gemisch die Mischkammer verlässt;
ein Ventil (36), das an die Gaszufuhr anschließbar ist und einem Benutzer ermöglicht, die Strömung des Gases von der Gaszufuhr zu der Vorrichtung (10) selektiv einzustellen; und
einen Massenströmungssensor (92), der zwischen dem Strömungssteuerventil (36) und der Gaseinlassöffnung (66) der Mischkammer (60) zum Messen des Massendurchsatzes des in die Mischkammer (60) strömenden Gases angeordnet ist;
**dadurch gekennzeichnet, dass** die Vorrichtung ferner Folgendes aufweist:
ein motorisiertes Gebläse (94) zum Bewegen des Gas-/Luft-Gemisches aus der Mischkammer (60) hinaus zu einer Stelle, an der das Gas-/Luft-Gemisch vom Benutzer geschnüffelt werden kann, um Geruch festzustellen;
eine Steuerung (102) zum Halten des motorisierten Gebläses (94) auf einer im Wesentlichen konstanten bestimmten Geschwindigkeit;
einen Prozessor;
eine Tastatur (42) für die Eingabe von Daten, die zu einem bestimmten Test in Beziehung stehen;
eine Anzeige (32) zum Anzeigen von Daten, die zu einem bestimmten Test in Beziehung stehen;
einen nichtflüchtigen Speicher (106), der mit dem Prozessor betriebsmäßig gekoppelt ist, um die zu dem bestimmten Test in Beziehung stehenden Daten zu speichern, wobei die Daten eines oder mehrere beinhalten von: einem Geruchsnachweispegel, einem Bedienungsperson-Identifikator, einem Datum und einem Zeitstempel des Tests, einem Testort, einer Testzeit, von der Bedienungsperson eingegebenen Anmerkungen und einer Anzeige von jeglichen Testfehlern; und
eine Programmeinrichtung, die von dem Prozessor ausführbar ist, um den Zugriff auf die in dem nichtflüchtigen Speicher gespeicherten Daten für einen Benutzer zu begrenzen.

2. Tragbare Vorrichtung nach Anspruch 1,
wobei die Steuerung (102) einen Drehzahlmesser (97) zum Bestimmen der Drehzahl des motorisierten Gebläses (94) aufweist.

3. Tragbare Vorrichtung nach Anspruch 1 oder 2,
weiterhin aufweisend ein Infrarot-Sender-und-Empfänger-Paar (96), das auf entgegengesetzten Seiten des motorisierten Gebläses (94) angeordnet ist, um einen Infrarotstrahl zu erzeugen, der von der Bewegung von Schaufeln des motorisierten Gebläses (94) unterbrochen werden kann.

4. Tragbare Vorrichtung nach Anspruch 2 oder 3,
weiterhin aufweisend ein Mischelement (64) an der Lufteinlassöffnung (48) der Mischkammer (60), um eine Luftströmungsturbulenz zum Unterstützen des Mischens der Luft und des Gases hervorzurufen.

5. Tragbare Vorrichtung nach Anspruch 4,
wobei das Mischelement (64) ein stationäres Gebläse aufweist.

6. Tragbare Vorrichtung nach einem der vorausgehenden Ansprüche,
wobei sich das motorisierte Gebläse (94) in einem Kanal befindet, der mit der Auslassöffnung der Mischkammer (60) in Verbindung steht, um das Gas-/Luft-Gemisch aus der Mischkammer abzuziehen.

7. Tragbare Vorrichtung nach einem der vorausgehenden Ansprüche,
weiterhin aufweisend eine Einrichtung zum Linearisieren von Massendurchsatz-Daten, die von dem Massenströmungssensor (92) erzeugt werden.

8. Tragbare Vorrichtung nach einem der vorausgehenden Ansprüche,
weiterhin aufweisend eine Batterie (16) für die Energieversorgung der Vorrichtung.

9. Tragbare Vorrichtung nach Anspruch 8,
wobei die Batterie (16) wiederaufladbar ist.

10. Tragbare Vorrichtung nach Anspruch 9,
weiterhin aufweisend einen mit der Batterie (16) verbundenen Ladeanschluss, um ein Wiederaufladen der Batterie vor Ort zu ermöglichen.

11. Tragbare Vorrichtung nach Anspruch 10,
weiterhin aufweisend eine Batteriekompensations-Schaltungseinrichtung (112), um dem Massenströmungssensor (92) ein exaktes Arbeiten innerhalb eines bestimmten Spannungsbereichs der Batterie (16) zu ermöglichen.

12. Tragbare Vorrichtung nach einem der vorausgehenden Ansprüche,
weiterhin aufweisend eine Einrichtung zum Verbinden der Vorrichtung (10) mit einem Computer (108), um Daten zu dem Computer zu übermitteln sowie Daten von dem Computer zu empfangen.

13. Tragbare Vorrichtung nach einem der vorausgehenden Ansprüche,
weiterhin aufweisend eine Einrichtung zum Verbinden der Vorrichtung (10) mit einem Drucker zum Ausdrucken von Daten von der Vorrichtung.

14. Tragbare Vorrichtung nach Anspruch 12,
wobei der Computer (108) eine Einrichtung zum Verarbeiten und Anzeigen der mit dem bestimmten Test in Beziehung stehenden Daten aufweist.

15. Tragbare Vorrichtung nach Anspruch 14,
wobei die Einrichtung zum Verarbeiten von Daten eine Einrichtung zum Erstellen von Grafiken aus den Daten aufweist.

16. Tragbare Vorrichtung nach Anspruch 14,
wobei die Einrichtung zum Verarbeiten von Daten eine Einrichtung zum Filtern der Daten aufweist.

17. Tragbare Vorrichtung nach Anspruch 12,
wobei der Computer eine Einrichtung zum Archivieren der mit dem bestimmten Test in Beziehung stehenden Daten aufweist.

## Revendications

1. Appareil portatif (10) permettant de déterminer des niveaux d'odeur dans des courants gazeux, comprenant :
une chambre de mélange (60) pour mélanger un gaz provenant d'une alimentation en gaz avec l'air ambiant pour former un mélange gaz-air, ladite chambre de mélange (60) incluant une lumière d'entrée de gaz (66) à travers laquelle le gaz est reçu, une lumière d'admission d'air (48) à travers laquelle l'air est reçu, et une lumière de refoulement à travers laquelle le mélange gaz-air sort de ladite chambre de mélange ;
une soupape (36) pouvant être raccordée à l'alimentation en gaz, permettant à un utilisateur de régler sélectivement l'écoulement du gaz de l'alimentation en gaz vers l'appareil (10) ; et
un capteur de débit massique (92) positionné entre la soupape de régulation d'écoulement (36) et la lumière d'entrée de gaz (66) de ladite chambre de mélange (60) pour mesurer le débit massique du gaz en écoulement dans la chambre de mélange (60) ; **caractérisé en ce que** l'appareil comprend en outre :
un ventilateur motorisé (94) pour déplacer le mélange gaz-air hors de la chambre de mélange (60) vers un emplacement où ledit mélange gaz-air peut être reniflé par l'utilisateur pour détecter une odeur ;
une unité de commande (102) pour maintenir ledit ventilateur motorisé (94) à une vitesse donnée sensiblement constante ;
une unité de traitement ;
un pavé numérique (42) pour l'entrée de données associées à un essai donné ;
un affichage (32) pour afficher des données associées à un essai donné ;
une mémoire non volatile (106) couplée fonctionnellement à l'unité de traitement pour stocker les données associées à l'essai donné, les données incluant un ou plusieurs des éléments suivants : un niveau de détection d'odeur, un identifiant d'opérateur, une date et une estampille temporelle de l'essai, un emplacement d'essai, une heure d'essai, des notes entrées par l'opérateur, et une indication d'erreurs d'essai ; et
un moyen de programme exécutable par l'unité de traitement pour restreindre l'accès par un utilisateur aux données stockées dans la mémoire non volatile.

2. Appareil portatif selon la revendication 1, dans lequel ladite unité de commande (102) inclut un tachymètre (97) pour déterminer une vitesse de rotation du ventilateur motorisé (94).

3. Appareil portatif selon la revendication 1 ou 2, comprenant en outre une paire d'émetteur et récepteur infrarouge (96) positionnée sur des côtés opposés dudit ventilateur motorisé (94) pour générer un faisceau infrarouge qui peut être interrompu par le mouvement de pales dudit ventilateur motorisé (94).

4. Appareil portatif selon la revendication 1, 2 ou 3, comprenant en outre un élément de mélange (64) au niveau de ladite lumière d'admission d'air (48) de ladite chambre de mélange (60) pour provoquer une turbulence d'écoulement d'air afin de favoriser un mélange dudit air et dudit gaz.

5. Appareil portatif selon la revendication 4, dans lequel l'élément de mélange (64) comprend un ventilateur stationnaire.

6. Appareil portatif selon l'une quelconque des revendications précédentes, dans lequel ledit ventilateur motorisé (94) est situé dans un conduit raccordé à la lumière de refoulement de la chambre de mélange (60) pour aspirer le mélange gaz-air hors de ladite chambre de mélange.

7. Appareil portatif selon l'une quelconque des revendications précédentes, comprenant en outre un moyen pour linéariser des données de débit massique générées par le capteur de débit massique (92).

8. Appareil portatif selon l'une quelconque des revendications précédentes, comprenant en outre une batterie (16) pour alimenter l'appareil.

9. Appareil portatif selon la revendication 8, dans lequel ladite batterie (16) est rechargeable.

10. Appareil portatif selon la revendication 9, comprenant en outre une lumière de chargeur connectée à ladite batterie (16) pour permettre à ladite batterie d'être rechargée in situ.

11. Appareil portatif selon la revendication 10, comprenant en outre des circuits de compensation de batterie (112) pour permettre audit capteur de débit massique (92) de fonctionner avec précision dans une plage de tensions donnée de ladite batterie (16).

12. Appareil portatif selon l'une quelconque des revendications précédentes, comprenant un moyen pour relier ledit appareil (10) à un ordinateur (108) pour transmettre des données audit ordinateur et recevoir des données de celui-ci.

13. Appareil portatif selon l'une quelconque des revendications précédentes, comprenant en outre un moyen pour relier ledit appareil (10) à une imprimante pour imprimer des données à partir dudit appareil.

14. Appareil portatif selon la revendication 12, dans lequel l'ordinateur (108) inclut un moyen pour traiter et afficher des données associées à l'essai donné.

15. Appareil portatif selon la revendication 14, dans lequel ledit moyen pour traiter des données inclut un moyen pour générer des graphiques à partir desdites données.

16. Appareil portatif selon la revendication 14, dans lequel ledit moyen pour traiter des données inclut un moyen pour filtrer lesdites données.

17. Appareil portatif selon la revendication 12, dans lequel ledit ordinateur inclut un moyen pour archiver les données associées à l'essai donné.
